# EUROPÄISCHE PATENTSCHRIFT

(11) **EP 0 769 494 B1**
(45) Veröffentlichungstag und Bekanntmachung des Hinweises auf die Patenterteilung: **19.02.2003**
(21) Anmeldenummer: 96116283.1
(22) Anmeldetag: 11.10.1996
(51) Int. Cl.: C07D 235/26

(54) **Verfahren zur Aufarbeitung von Benzimidazolone enthaltenden Reaktionsgemischen**
Process for working up reaction mixtures containing benzimidazolones
Procédé de traitement d'un mélange réactionnel contenant des benzimidazoles

(30) Priorität: 20.10.1995 DE 19539114
(43) Veröffentlichungstag der Anmeldung: 23.04.1997
(73) Patentinhaber: Clariant GmbH, 65929 Frankfurt am Main (DE)
(72) Erfinder: Angenendt, Heinrich, Dr., 65510 Idstein (DE); Portz, Bert Willi, Dr., 65929 Frankfurt (DE); Walter, Horst, 65795 Hattersheim (DE)

(56) Entgegenhaltungen:
- DE-A- 2 725 957

## Beschreibung

Die vorliegende Erfindung betrifft ein gegenüber dem Stand der Technik verbessertes Verfahren zur Aufarbeitung von durch Umsetzung von o-Phenylendiaminen und Harnstoff in einem wasserunlöslichen Lösungsmittel hergestellte Benzimidazolone enthaltenden Reaktionsgemischen.

Benzimidazolone lassen sich, wie in der DE-OS 27 25 957 beschrieben, durch Umsetzung eines o-Phenylendiamins mit Harnstoff in einem organischen Lösungsmittel bei einer Temperatur von 100 bis 200°C unter Abspaltung von Ammoniak herstellen. Das verwendete Lösungsmittel soll einen Siedepunkt von über 100°C haben und eine Löslichkeit in Wasser von nicht mehr als 5 Gramm pro 1 Liter Wasser aufweisen.

Nachteilig an diesem Verfahren ist die technisch aufwendige Aufarbeitung der dabei anfallenden Reaktionsgemische. Wie aus den Beispielen der DE-OS 27 25 957 hervorgeht, wird das Reaktionsgemisch, das eine Suspension des Benzimidazolons in dem organischen Lösungsmittel darstellt, filtriert, das abfiltrierte Benzimidazolon zunächst mit Ethanol oder wäßrigem Ethanol und gegegenenfalls mit Wasser gewaschen und im Vakuum getrocknet. Diese Aufarbeitung ist technisch aufwendig, da zum einen mehrere Arbeitsschritte erforderlich sind und zum anderen das in der Reaktion verwendete Lösungsmittel und das in der Aufarbeitung anfallende wäßrige Ethanol aufwendig aufgearbeitet werden müssen. Verzichtet man auf die Wäsche mit Ethanol oder wäßrigem Ethanol und gegebenenfalls Wasser, so muß das Benzimidazolon unter einem erheblichen Aufwand an Zeit und Energie vom Lösungsmittel befreit werden. Darüber hinaus fällt wegen der fehlenden Wäschen ein Benzimidazolon in minderwertiger Qualität an.

Abweichend von der Lehre der DE-OS 27 25 957 könnte man in Betracht ziehen, das Lösungsmittel mit Wasser azeotrop abzudestillieren. Versucht man jedoch, das Lösungsmittel mit Wasser azeotrop auszukreisen, so tritt bereits bei Beginn der Destillation ein starkes Schäumen auf und zum Ende der Destillation beginnt das Benzimidazolon unter beträchtlicher Vergrößerung des beanspruchten Volumens auf dem Wasser aufzuschwimmen. Beide Effekte, Schäumen und Aufschwimmen, stellen eine erhebliche Beeinträchtigung dar und könnten nur durch eine signifikante Vergrößerung des Reaktorvolumens beherrscht werden. Eine derartige Arbeitsweise würde jedoch kaum lösbare Probleme nach sich ziehen, da einerseits entsprechend überdimensionierte Reaktoren üblicherweise nicht zur Verfügung stehen und zum anderen die Handhabung relativ kleiner Mengen von Reaktionsgemischen in Reaktoren, die im Vergleich zum anfallenden Reaktionsgemisch sehr große Volumina aufweisen, zu technischen Schwierigkeiten führt.

Es besteht also ein Bedarf nach einem Aufarbeitungsverfahren, das die vorstehend genannten Nachteile vermeidet und sich zudem auf einfache Art und Weise und ohne großen technischen Aufwand realisieren läßt. Ferner soll das Wertprodukt in hoher Ausbeute und zugleich in hoher Reinheit gewonnen werden.

Gelöst wird diese Aufgabe durch ein Verfahren zur Aufarbeitung von durch Umsetzung von o-Phenylendiaminen und Harnstoff in einem wasserunlöslichen Lösungsmittel hergestellte Benzimidazolone der Formel (I) 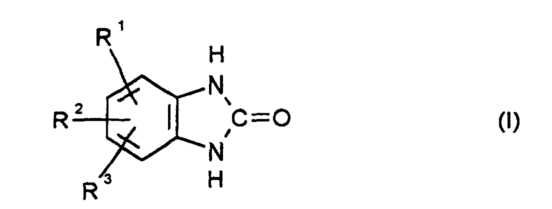 enthaltenden Reaktionsgemischen, wobei in Formel (I) R¹, R² und R³ gleich oder verschieden sind und unabhängig voneinander für ein Wasserstoffatom, eine Alkyl- oder Alkoxygruppe mit jeweils 1 bis 4 Kohlenstoffatomen, Chlor, eine Hydroxy-, Cyan- oder Nitrogruppe stehen. Es ist dadurch gekennzeichnet, daß man dem Reaktionsgemisch ein Netzmittel und einen Entschäumer zusetzt, das Lösungsmittel unter Zugabe von Wasser und unter Rühren azeotrop abdestilliert und aus der anfallenden wäßrigen Suspension das Benzimidazolon abfiltriert.

Unter Netzmittel versteht man grenzflächenaktive Verbindungen, die die Grenzflächenspannung von Flüssigkeiten herabsetzen, wodurch sich die Flüssigkeit auf Oberflächen besser ausbreitet und diese benetzt.

Entschäumer (Antischaummittel) sind in der schäumenden Flüssigkeit schwer lösliche, oberflächenaktive Stoffe, die unter Filmbildung die Schaumbildner von der Oberfläche verdrängen und dadurch die Schaumbildung vermindern oder unterdrücken, oder Stoffe, die die Oberflächenspannung des Wassers erhöhen.

Setzt man dem Reaktionsgemisch nur ein Netzmittel zu, so wird zwar das Aufschwimmen des Benzimidazolons am Ende der Destillation verhindert, jedoch gleichzeitig das Schäumen unkontrollierbar verstärkt. Setzt man hingegen nur einen Entschäumer zu, so wird zwar das Schäumen zu Beginn und während der Destillation verhindert, man erhält aber am Ende der Azeotropdestillation ein verklebtes, nicht filtrierbares, aufgeblähtes Reaktionsgemisch, in dem das Benzimidazolon enthalten ist.

Erst die gleichzeitige Verwendung von Netzmittel und Entschäumer führt zum gewünschten Erfolg und verhindert das unerwünschte Schäumen zu Beginn und während der Destilllation und das unerwünschte Aufschwimmen des Benzimidazolons am Ende der Destillation. Dies ist überraschend, da die Eigenschaften des Netzmittels und des Entschäumers einander entgegengesetzt sind und man erwarten würde, daß sie sich in ihrer Wirkung gegenseitig kompensieren würden. Dies ist jedoch unerwarteterweise nicht der Fall.

Ein weiterer Vorteil des erfindungsgemäßen Verfahrens ist, daß das ausgekreiste Lösungsmittel in derart reiner Form anfällt, daß es ohne weitere Aufarbeitung wieder in das Verfahren eingesetzt werden kann. Auch das Abwasser weist lediglich eine geringe Belastung auf und stellt somit kein besonderes Problem dar.

Das Verfahren eignet sich ebenfalls dafür, ein Reaktionsgemisch, das ein Benzimidazolon der Formel (I), worin R¹, R² und R³ für ein Wasserstoffatom stehen, also ein Reaktionsgemisch, das ein unsubstituiertes Benzimidazolon enthält, einzusetzen.

Man kann in das Verfahren mit gutem Erfolg übliche Netzmittel, beispielsweise ein Alkylsulfonat mit 12 bis 18 Kohlenstoffatomen oder ein Gemisch derartiger Alkylsulfonate, insbesondere ein sekundäres lineares Alkylsulfonat mit 12 bis 18 Kohlenstoffatomen oder ein Gemisch derartiger Alkylsulfonate einsetzen.

Man kann aber auch als Netzmittel ein Paraffinsulfonat mit 14 bis 17 Kohlenstoffatomen oder ein Gemisch derartiger Paraffinsulfonate oder Alkylbenzolsulfonate mit 10 bis 13 Kohlenstoffatomen oder ein Gemisch derartiger Alkylbenzolsulfonate einsetzen.

Als Entschäumer lassen sich übliche Entschäumer verwenden. Man kann mit gutem Erfolg als Entschäumer ein Trialkylphosphat mit 3 bis 20 Kohlenstoffatomen je Alkylrest oder ein Gemisch derartiger Trialkylphosphate, insbesondere Tri-n-butylphosphat einsetzen.

Als Entschäumer kann man ebenfalls einen ein Fett oder Öl, einen Ester höherer Carbonsäuren, einen höheren Alkohol, ein Polyalkylenglykol, Polyglykol, Silikon als oberflächenaktiven Stoff oder Mischungen derselben enthaltenden Entschäumer, insbesondere einen, einen Ester höherer Carbonsäuren, einen höheren Alkohol, ein Polyalkylenglykol, Polyglykol als oberflächenaktiven Stoff oder Mischungen derselben enthaltenden Entschäumer einsetzen.

Nachdem man dem Reaktionsgemisch das Netzmittel und den Entschäumer zugesetzt hat, destilliert man anschließend das Lösungsmittel unter Zugabe von Wasser ab. Dabei kann man das Wasser in flüssiger Form oder in Form von Wasserdampf dem Reaktionsgemisch zusetzen. In einer Reihe von Fällen kann es günstig sein, das Lösungsmittel unter Zugabe von Wasser in Form von Wasserdampf azeotrop abzudestillieren. Es ist jedoch auch möglich, das Wasser in flüssiger Form dem Reaktionsgemisch kontinuierlich oder diskontinuierlich zuzusetzen. Man kann aber auch Wasser in flüssiger Form und Wasserdampf zugleich verwenden.

Man setzt üblicherweise 0,1 bis 5, insbesondere 0,25 bis 2,5, bevorzugt 0,4 bis 1 Gew.-%, Netzmittel, bezogen auf Benzimidazolon, ein.

Im allgemeinen genügt es, 0,1 bis 5, insbesondere 0,25 bis 2,5, bevorzugt 0,4 bis 1 Gew.-%, Entschäumer, bezogen auf Benzimidazolon, einzusetzen.

Falls erforderlich, kann das Netzmittel ebenso wie der Entschäumer auch in größeren Mengen, beispielsweise bis 10 Gew.-% und mehr, bezogen auf Benzimidazolon, zum Einsatz gelangen. In der Mehrzahl der Fälle kann man sich jedoch mit den vorstehend genannten Mengen begnügen, ohne eine merkliche Beeinträchtigung des Verfahrens in Kauf nehmen zu müssen.

Es versteht sich von selbst, daß sowohl die Art des Benzimidazolons als auch die Art des jeweils verwendeten Netzmittels und Entschäumers den Mengenbedarf an Netzmittel und Entschäumer in gewissem Umfang beeinflussen können. Dies ist gegebenenfalls entsprechend zu berücksichtigen.

Bei der Herstellung des Benzimidazolons der Formel (I) setzt man als Lösungsmittel ein organisches Lösungsmittel, das in Wasser nicht löslich ist, beispielsweise Xylol, Chlorbenzol, 1,2-Dichlorbenzol, 1,3-Dichlorbenzol, 1,4-Dichlorbenzol, ein Gemisch isomerer Dichlorbenzole, 2-Chlortoluol, 3-Chlortoluol, 4-Chlortoluol, ein Gemisch isomerer Chlortoluole, insbesondere 1,2-Dichlorbenzol, 2-Chlortoluol oder ein Gemisch isomerer Chlortoluole ein.

In einer Reihe von Fällen kann es günstig sein, das Reaktionsgemisch mit Hilfe eines radial wirkenden Rührers zu rühren, um ein Aufschäumen während der azeotropen Destillation gering zu halten und auf diese Weise die Wirkung des Netzmittels und des Entschäumers zu unterstützen.

Das erfindungsgemäße Verfahren läßt sich sowohl bei Unterdruck, als auch bei Normaldruck und Überdruck ausführen. Man kann es sowohl kontinuierlich als auch diskontinuierlich ausführen. Es eignet sich besonders gut für eine diskontinuierliche Verfahrensdurchführung.

Das nach Filtration anfallende, wasserfeuchte Benzimidazolon der Formel (I) kann in einer Vielzahl von Fällen ohne zusätzliche Aufarbeitung weiterverarbeitet werden.

Die nachfolgenden Beispiele beschreiben die Erfindung näher, ohne sie darauf zu beschränken:

### Experimenteller Teil:

### Vergleichsbeispiel 1

### Azeotrope Destillation ohne Netzmittel und ohne Entschäumer

In einem 3,0 I Rührkolben mit Wasserabscheider und Rückflußkühler werden 273 g Phenylendiamin und 155 g Harnstoff in 715 ml 2-Chlortoluol auf 120 bis 160°C bis zum Ende der Ammoniakentwicklung erhitzt. Anschließend kühlt man auf etwa 90°C ab und kreist unter kontinuierlicher Zugabe von insgesamt 930 ml Wasser das 2-Chlortoluol vorsichtig azeotrop aus. Anschließend kühlt man auf Raumtemperatur ab, saugt den Feststoff ab und erhält nach Waschen mit insgesamt 1000 ml Wasser 389 g Benzimidazolon mit einem Wassergehalt von 15 %. Dies entspricht einer Ausbeute von 99 % der Theorie. Zu Anfang der Azeotropdestillation kommt es zu starkem Schäumen, wobei sich das Volumen des Reaktionsgemisches von zunächst etwa 1000 ml auf etwa 1700 ml vergrößert. In der Endphase der Azeotropdestillation beginnt das Benzimidazolon aufzuschwimmen, wobei sich das Volumen des Reaktionsgemisches auf 2500 bis 3000 ml vergrößert und das Reaktionsgemisch zeitweise bis in den Kühler schäumt.

### Vergleichsbeispiel 2

### Azeotrope Destillation mit Netzmittel, aber ohne Entschäumer

In einem 3,0 I Rührkolben mit Wasserabscheider und Rückflußkühler werden 273 g Phenylendiamin und 155 g Harnstoff in 715 ml 2-Chlortoluol auf 120 bis 160°C bis zum Ende der Ammoniakentwicklung erhitzt. Anschließend kühlt man auf etwa 90°C ab und gibt 1,8 g Hostapur SAS 60 (60 %ige wäßrige Lösung sekundärer linearer Alkansulfonate (C₁₂-C₁₈), ein Handelsprodukt der Hoechst Aktiengesellschaft) zu. Dann kreist man unter kontinuierlicher Zugabe von Wasser das 2-Chlortoluol aus. Nach Auskreisen von etwa 600 ml 2-Chlortoluol und Zugabe von etwa 780 ml Wasser beginnt das Gemisch unkontrollierbar zu schäumen. Der Versuch muß deshalb abgebrochen werden.

### Vergleichsbeispiel 3

### Azeotrope Destillation ohne Netzmittel, aber mit Entschäumer

In einem 3,0 I Rührkolben mit Wasserabscheider und Rückflußkühler werden 273 g Phenylendiamin und 155 g Harnstoff in 715 ml 2-Chlortoluol auf 120 bis 160°C bis zum Ende der Ammoniakentwicklung erhitzt. Anschließend kühlt man auf etwa 90°C ab und gibt 1,8 g Tri-n-butylphosphat zu. Dann kreist man unter kontinuierlicher Zugabe von Wasser das 2-Chlortoluol aus. Nach Auskreisen von etwa 500 ml 2-Chlortoluol und Zugabe von ca. 650 ml Wasser beginnt das Reaktionsgemisch immer zäher zu werden, wobei das Volumen des Gemisches auf etwa 2000 bis 2500 ml ansteigt. Das verklebte Benzimidazolon kann nur mit sehr viel Wasser aus dem Kolben ausgespült werden.

### Beispiel 1

### Azeotrope Destillation mit Netzmittel und mit Entschäumer

In einem 3,0 I Rührkolben mit Blattrührer, Wasserabscheider und Rückflußkühler werden 273 g Phenylendiamin und 155 g Harnstoff in 715 ml 2-Chlortoluol auf 120 bis 160°C bis zum Ende der Ammoniakentwicklung erhitzt. Anschließend kühlt man auf < 90°C ab, gibt 1,8 g Marlon A350 (Gemisch von Alkylbenzolsulfonaten (C₁₀-C₁₃), ein Handelsprodukt von Hüls) und 1,8 g Entschäumer FN (eine fluorhaltige, nichttensidische Mischung verschiedener Polyalkylenglykolderivate; ein Handelsprodukt der Hoechst Aktiengesellschaft) zu und kreist anschließend unter kontinuierlicher Zugabe von insgesamt 930 ml Wasser das 2-Chlortoluol aus. Anschließend kühlt man auf Raumtemperatur ab, saugt den Feststoff ab und erhält nach Waschen mit insgesamt 1000 ml Wasser 394 g Benzimidazolon mit einem Wassergehalt von 16 %, dies entspricht einer Ausbeute von 99 % der Theorie.
Während der Azeotropdestillation vergrößert sich das Volumen des Reaktionsgemisches von zunächst etwa 1000 ml auf lediglich 1300 bis 1400 ml, wobei nur ein leichtes Schäumen zu beobachten ist.

### Beispiel 2

### Azeotrope Destillation mit Netzmittel und mit Entschäumer

In einem 3,0 I Rührkolben mit Blattrührer, Wasserabscheider und Rückflußkühler werden 383 g 4-Nitro-1,2-phenylendiamin und 155 g Harnstoff in 900 ml 2-Chlortoluol auf 120 bis 160°C bis zum Ende der Ammoniakentwicklung erhitzt. Anschließend kühlt man auf < 90°C ab, gibt 1,8 g Hostapur SAS 60 (eine 60 %ige wäßrige Lösung eines Gemisches sekundärer linearer Alkansulfonate (C₁₂-C₁₈), ein Handelsprodukt der HOECHST Aktiengesellschaft) und 1,8 g Tri-n-butylphosphat zu und kreist anschließend unter kontinuierlicher Zugabe von insgesamt 1100 ml Wasser das 2-Chlortoluol aus, wobei sich das Reaktionsvolumen um ca. 30 bis 40 % vergrößert. Anschließend kühlt man auf Raumtemperatur ab, saugt den Feststoff ab und erhält nach Waschen mit insgesamt 1000 ml Wasser und Trocknen im Vakuum 425 g 4-Nitro-2-benzimidazolon. Dies entspricht einer Ausbeute von 95 % der Theorie.

### Beispiel 3

### Azeotrope Destillation mit Netzmittel und mit Entschäumer

In einem 3,0 I Rührkolben mit Blattrührer, Wasserabscheider und Rückflußkühler werden 273 g Phenylendiamin und 155 g Harnstoff in 715 ml 2-Chlortoluol auf 120 bis 160°C bis zum Ende der Ammoniakentwicklung erhitzt. Anschließend kühlt man auf < 90°C ab, gibt 1,8 g Marlon PS (ein Gemisch von Paraffinsulfonaten (C₁₄-C₁₇), ein Handelsprodukt von Hüls) und 1,8 g Entschäumer FN zu und kreist anschließend unter kontinuierlicher Zugabe von insgesamt 930 ml Wasser das 2-Chlortoluol aus. Anschließend kühlt man auf Raumtemperatur ab, saugt den Feststoff ab und erhält nach Waschen mit insgesamt 1000 ml Wasser 403 g Benzimidazolon mit einem Wassergehalt von 18 %. Dies entspricht einer Ausbeute von 99 % der Theorie.
Während der Azeotropdestillation vergrößert sich das Volumen des Reaktionsgemisches von zunächst etwa 1000 ml auf 1300 bis 1400 ml, wobei nur ein leichtes Schäumen zu beobachten ist.

### Beispiel 4

### Azeotrope Destillation mit Netzmittel und mit Entschäumer

In einem 3,0 I Rührkolben mit Blattrührer, Wasserabscheider und Rückflußkühler werden 273 g Phenylendiamin und 155 g Harnstoff in 715 ml 2-Chlortoluol auf 120 bis 160°C bis zum Ende der Ammoniakentwicklung erhitzt. Anschließend kühlt man auf < 90°C ab, gibt 1,8 g Hostapur SAS 60 (eine 60 %ige wäßrige Lösung eines Gemisches sekundärer linearer Alkansulfonate (C₁₂-C₁₈)) und 1,8 g Entschäumer (Entschäumer auf Basis Ester höherer Carbonsäuren; ein Handelsprodukt der Hoechst Aktiengesellschaft) zu und kreist anschließend unter kontinuierlicher Zugabe von insgesamt 930 ml Wasser das 2-Chlortoluol aus. Anschließend kühlt man auf Raumtemperatur ab, saugt den Feststoff ab und erhält nach Waschen mit insgesamt 1000 ml Wasser 380 g Benzimidazolon mit einem Wassergehalt von 13 %. Dies entspricht einer Ausbeute von 99 % der Theorie.
Während der Azeotropdestillation vergrößert sich das Volumen des Reaktionsgemisches von zunächst etwa 1000 ml auf 1300 bis 1400 ml, wobei nur ein leichtes Schäumen zu beobachten ist.

### Beispiel 5

### Azeotrope Destillation zusätzlich mit radial wirkendem Rührer

In einem 30 I Rührkolben mit radial wirkendem Blattrührer, Wasserabscheider und Rückflußkühler werden 3630 g o-Phenylendiamin und 2064 g Harnstoff in 10 I 2-Chlortoluol auf 120 bis 160°C bis zum Ende der Ammoniakentwicklung erhitzt. Anschließend kühlt man auf < 90°C ab, gibt 24 g Hostapur SAS 60 (60%ige wäßrige Lösung sekundärer linearer Alkansulfonate (C₁₂-C₁₈)) und 24 g Tri-n-butylphosphat zu und kreist anschließend unter kontinuierlicher Zugabe von insgesamt 12 I Wasser das 2-Chlortoluol aus. Anschließend kühlt man auf Raumtemperatur ab, saugt den Feststoff ab und erhält nach Waschen mit insgesamt 10 I Wasser 5245 g Benzimidazolon mit einem Wassergehalt von 15 %. Dies entspricht einer Ausbeute von 99 % der Theorie. Während der Azeotropdestillation vergrößert sich das Volumen des Reaktionsgemisches von zunächst ca. 14 l auf 18 bis 19 I, wobei nur ein leichtes Schäumen zu beobachten ist. Analoge Ergebnisse erhält man mit Xylol oder Chlorbenzol als Lösungsmittel.

## Patentansprüche

1. Verfahren zur Aufarbeitung von durch Umsetzung von o-Phenylendiaminen und Harnstoff in einem wasserunlöslichen Lösungsmittel hergestellte Benzimidazolone der Formel (I) 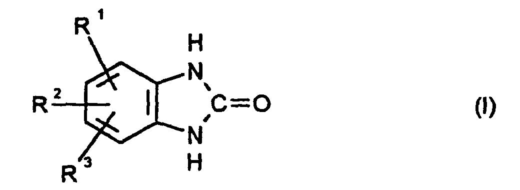 enthaltenden Reaktionsgemischen, wobei in Formel (I) R¹, R² und R³ gleich oder verschieden sind und unabhängig voneinander für ein Wasserstoffatom, eine Alkyl- oder Alkoxygruppe mit jeweils 1 bis 4 Kohlenstoffatomen, Chlor, eine Hydroxy-, Cyan oder Nitrogruppe stehen, **dadurch gekennzeichnet, daß** man dem Reaktionsgemisch ein Netzmittel und einen Entschäumer zusetzt, das Lösungsmittel unter Zugabe von Wasser und unter Rühren azeotrop abdestilliert und aus der anfallenden wäßrigen Suspension das Benzimidazolon abfiltriert.

2. Verfahren nach Anspruch 1 **dadurch gekennzeichnet, daß** man ein Reaktionsgemisch einsetzt, das ein Benzimidazolon der Formel (I), worin zwei der Reste R¹, R² oder R³ für ein Wasserstoffatom stehen, enthält.

3. Verfahren nach Anspruch 1 oder 2, **dadurch gekennzeichnet, daß** man ein Reaktionsgemisch einsetzt, das ein Benzimidazolon der Formel (I), worin R¹, R² und R³ für ein Wasserstoffatom stehen, enthält.

4. Verfahren nach einem oder mehreren der Ansprüche 1 bis 3, **dadurch gekennzeichnet, daß** man als Netzmittel ein Alkylsulfonat mit 12 bis 18 Kohlenstoffatomen oder ein Gemisch derartiger Alkylsulfonate einsetzt.

5. Verfahren nach einem oder mehreren der Ansprüche 1 bis 4, **dadurch gekennzeichnet, daß** man als Netzmittel ein sekundäres lineares Alkylsulfonat mit 12 bis 18 Kohlenstoffatomen oder ein Gemisch derartiger Alkylsulfonate einsetzt.

6. Verfahren nach einem oder mehreren der Ansprüche 1 bis 5, **dadurch gekennzeichnet, daß** man als Netzmittel ein Paraffinsulfonat mit 14 bis 17 Kohlenstoffatomen oder ein Gemisch derartiger Paraffinsulfonate einsetzt.

7. Verfahren nach einem oder mehreren der Ansprüche 1 bis 6, **dadurch gekennzeichnet, daß** man als Netzmittel ein Alkylbenzolsulfonat mit 10 bis 13 Kohlenstoffatomen oder ein Gemisch derartiger Alkylbenzolsulfonate einsetzt.

8. Verfahren nach einem oder mehreren der Ansprüche 1 bis 7, **dadurch gekennzeichnet, daß** man als Entschäumer ein Trialkylphosphat mit 3 bis 20 Kohlenstoffatomen je Alkylrest oder ein Gemisch derartiger Trialkylphosphate einsetzt.

9. Verfahren nach einem oder mehreren der Ansprüche 1 bis 8, **dadurch gekennzeichnet, daß** man als Entschäumer Tri-n-butylphosphat einsetzt.

10. Verfahren nach einem oder mehreren der Ansprüche 1 bis 7, **dadurch gekennzeichnet, daß** man als Entschäumer einen ein Fett oder Öl, einen Ester höherer Carbonsäuren, einen höheren Alkohol, ein Polyalkylenglykol, Polyglykol, Silicon als oberflächenaktiven Stoff oder Mischungen derselben enthaltenden Entschäumer einsetzt.

11. Verfahren nach einem oder mehreren der Ansprüche 1 bis 7, **dadurch gekennzeichnet, daß** man als Entschäumer einen Ester höherer Carbonsäuren, einen höheren Alkohol, ein Polyalkylenglykol, Polyglykol als oberflächenaktiven Stoff oder Mischungen derselben enthaltenden Entschäumer einsetzt.

12. Verfahren nach einem oder mehreren der Ansprüche 1 bis 11, **dadurch gekennzeichnet, daß** man 0,1 bis 5 Gew.-% Netzmittel, bezogen auf Benzimidazolon, einsetzt.

13. Verfahren nach einem oder mehreren der Ansprüche 1 bis 12, **dadurch gekennzeichnet, daß** man 0,1 bis 5 Gew.-% Entschäumer, bezogen auf Benzimidazolon, einsetzt.

14. Verfahren nach einem oder mehreren der Ansprüche 1 bis 13, **dadurch gekennzeichnet, daß** man das Lösungsmittel unter Zugabe von Wasser in Form von Wasserdampf azeotrop abdestilliert.

15. Verfahren nach einem oder mehreren der Ansprüche 1 bis 14, **dadurch gekennzeichnet, daß** man als Lösungsmittel 1,2-Dichlorbenzol, 2-Chlortoluol oder ein Gemisch isomerer Chlortoluole einsetzt.

16. Verfahren nach einem oder mehreren der Ansprüche 1 bis 15, **dadurch gekennzeichnet, daß** man mit Hilfe eines radial wirkenden Rührers rührt.

## Claims

1. A process for working up reaction mixtures containing benzimidazolones of the formula (I) 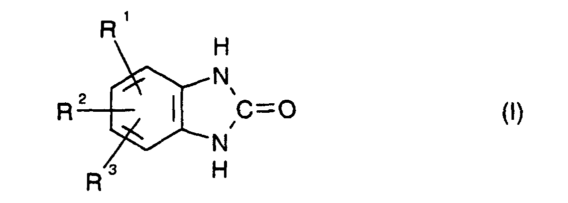 prepared by reacting o-phenylenediamines and urea in a water-insoluble solvent, where in formula (I) R¹, R² and R³ are identical or different and independently of one another are a hydrogen atom, an alkyl group or alkoxy group having in each case 1 to 4 carbon atoms, chlorine, a hydroxyl group, cyano group or nitro group, which comprises adding a wetting agent and a defoamer to the reaction mixture, distilling off the solvent azeotropically with the addition of water and with stirring and filtering off the benzimidazolone from the aqueous suspension arising.

2. The process as claimed in claim 1, wherein a reaction mixture is used which contains a benzimidazolone of the formula (I), in which two of the radicals R¹, R² or R³ are hydrogen atoms.

3. The process as claimed in claim 1 or 2, wherein a reaction mixture is used which contains a benzimidazolone of the formula (I), in which R¹, R² and R³ are hydrogen atoms.

4. The process as claimed in one or more of claims 1 to 3, wherein, as wetting agent, use is made of an alkylsulfonate having 12 to 18 carbon atoms or a mixture of such alkylsulfonates.

5. The process as claimed in one or more of claims 1 to 4, wherein, as wetting agent, use is made of a secondary linear alkylsulfonate having 12 to 18 carbon atoms or a mixture of such alkylsulfonates.

6. The process as claimed in one or more of claims 1 to 5, wherein, as wetting agent, use is made of a paraffinsulfonate having 14 to 17 carbon atoms or a mixture of such paraffinsulfonates.

7. The process as claimed in one or more of claims 1 to 6, wherein, as wetting agent, use is made of an alkylbenzenesulfonate having 10 to 13 carbon atoms or a mixture of such alkylbenzenesulfonates.

8. The process as claimed in one or more of claims 1 to 7, wherein, as defoamer, use is made of a trialkyl phosphate having 3 to 20 carbon atoms per alkyl radical or a mixture of such trialkyl phosphates.

9. The process as claimed in one or more of claims 1 to 8, wherein, as defoamer, use is made of tri-n-butyl phosphate.

10. The process as claimed in one or more of claims 1 to 7, wherein, as defoamer, use is made of a defoamer containing a fat or oil, an ester of higher carboxylic acids, a higher alcohol, a polyalkylene glycol, polyglycol, silicone as surface-active substance or mixtures of the same.

11. The process as claimed in one or more of claims 1 to 7, wherein, as defoamer, use is made of a defoamer containing an ester of higher carboxylic acids, a higher alcohol, a polyalkylene glycol, polyglycol as surface-active substance or mixtures of the same.

12. The process as claimed in one or more of claims 1 to 11, wherein 0.1 to 5% by weight of wetting agent is used, based on benzimidazolone.

13. The process as claimed in one or more of claims 1 to 12, wherein 0.1 to 5% by weight of defoamer is used, based on benzimidazolone.

14. The process as claimed in one or more of claims 1 to 13, wherein the solvent is distilled off azeoptrically with the addition of water in the form of steam.

15. The process as claimed in one or more of claims 1 to 14, wherein, as solvent, use is made of 1,2-dichlorobenzene, 2-chlorotoluene or a mixture of isomeric chlorotoluenes.

16. The process as claimed in one or more of claims 1 to 15, wherein the mixture is stirred using a radially acting agitator.

## Revendications

1. Procédé pour le traitement de mélanges réactionnels contenant des benzimidazolones de formule (I) 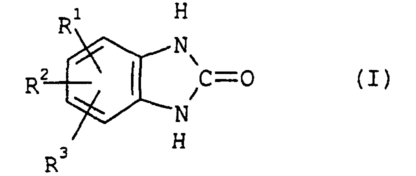 préparés par réaction d'o-phénylènediamines et d'urée dans un solvant insoluble dans l'eau, où, dans la formule (I), R¹, R² et R³ sont identiques ou différents et représentent chacun indépendamment de l'autre un groupe alkyle ou alcoxy ayant chacun de 1 à 4 atomes de carbone, un atome de chlore ou un groupe hydroxy, cyano ou nitro, **caractérisé en ce qu'**on ajoute au mélange réactionnel un mouillant et un antimousse, on chasse par distillation azéotropique le solvant par addition d'eau et sous agitation, et on isole la benzimidazolone par filtration à partir de la suspension aqueuse obtenue.

2. Procédé selon la revendication 1, **caractérisé en ce qu'**on utilise un mélange réactionnel qui contient une benzimidazolone de formule (I) dans laquelle deux des radicaux R¹, R² ou R³ sont des atomes d'hydrogène.

3. Procédé selon l'une des revendications 1 ou 2, **caractérisé en ce qu'**on utilise un mélange réactionnel qui contient une benzimidazolone de formule (I) dans laquelle R¹, R² et R³ sont des atomes d'hydrogène.

4. Procédé selon l'une ou plusieurs des revendications 1 à 3, **caractérisé en ce qu'**on utilise en tant que mouillant un alkylsulfonate ayant de 12 à 18 atomes de carbone ou un mélange d'alkylsulfonates de ce type.

5. Procédé selon l'une ou plusieurs des revendications 1 à 4, **caractérisé en ce qu'**on utilise en tant que mouillant un alkylsulfonate linéaire secondaire ayant de 12 à 18 atomes de carbone ou un mélange d'alkylsulfonates de ce type.

6. Procédé selon l'une ou plusieurs des revendications 1 à 5, **caractérisé en ce qu'**on utilise en tant que mouillant un paraffinesulfonate ayant de 14 à 17 atomes de carbone ou un mélange de paraffinesulfonates de ce type.

7. Procédé selon l'une ou plusieurs des revendications 1 à 6, **caractérisé en ce qu'**on utilise en tant que mouillant un alkylbenzènesulfonate ayant de 10 à 13 atomes de carbone ou un mélange d'alkylbenzènesulfonates de ce type.

8. Procédé selon l'une ou plusieurs des revendications 1 à 7, **caractérisé en ce qu'**on utilise en tant qu'antimousse un phosphate de trialkyle ayant de 3 à 20 atomes de carbone dans chaque fragment alkyle ou un mélange de phosphates de trialkyle de ce type.

9. Procédé selon l'une ou plusieurs des revendications 1 à 8, **caractérisé en ce qu'**on utilise en tant qu'antimousse du phosphate de tri-n-butyle.

10. Procédé selon l'une ou plusieurs des revendications 1 à 7, **caractérisé en ce qu'**on utilise en tant qu'antimousse un antimousse contenant une graisse ou une huile, un ester d'acides carboxyliques supérieurs, un alcool supérieur, un polyalkylèneglycol, un polyglycol, une silicone en tant que substance tensioactive, ou des mélanges de ceux-ci.

11. Procédé selon l'une ou plusieurs des revendications 1 à 7, **caractérisé en ce qu'**on utilise en tant qu'antimousse un antimousse contenant un ester d'acides carboxyliques supérieurs, un alcool supérieur, un polyalkylèneglycol, un polyglycol en tant que substance tensioactive, ou des mélanges de ceux-ci.

12. Procédé selon l'une ou plusieurs des revendications 1 à 11, **caractérisé en ce qu'**on utilise de 0,1 à 5 % en poids d'un mouillant par rapport à la benzimidazolone.

13. Procédé selon l'une ou plusieurs des revendications 1 à 12, **caractérisé en ce qu'**on utilise de 0,1 à 5 % en poids d'un antimousse par rapport à la benzimidazolone.

14. Procédé selon l'une ou plusieurs des revendications 1 à 13, **caractérisé en ce qu'**on chasse par distillation azéotropique le solvant par addition d'eau sous forme de vapeur d'eau.

15. Procédé selon l'une ou plusieurs des revendications 1 à 14, **caractérisé en ce qu'**on utilise en tant que solvant le 1,2-dichlorobenzène, le 2-chlorotoluène ou un mélange de chlorotoluènes isomères.

16. Procédé selon l'une ou plusieurs des revendications 1 à 15, **caractérisé en ce qu'**on agite à l'aide d'un agitateur à effet radial.
